# EUROPEAN PATENT APPLICATION

(11) **EP 4 052 695 A1**
(43) Date of publication of application: **07.09.2022**
(21) Application number: 21160914.4
(22) Date of filing: 05.03.2021
(51) Int. Cl.: A61K 9/00, A61K 9/20, A61K 31/00, A61K 31/4422

(54) **STABLE ORAL FIXED-DOSE IMMEDIATE RELEASE PHARMACEUTICAL COMPOSITIONS COMPRISING AMLODIPINE, ATORVASTATIN AND CANDESARTAN CILEXETIL**

(71) Applicant: Midas Pharma GmbH, 55218 Ingelheim (DE)
(72) Inventor: KAHM, Andreas, 55218 Ingelheim (DE); CADONAU, Stephanie, 55435 Gau-Algesheim (DE)
(74) Representative: Drescher, Christian

(57) **Abstract**

The present invention relates to stable oral fixed-dose immediate release pharmaceutical compositions comprising the active substances Amlodipine and Atorvastatin or their pharmaceutically acceptable salts or esters and Candesartan cilexetil that may be used for the treatment of hypertension and related diseases.

## Description

### DESCRIPTION OF THE INVENTION

The present invention relates to stable oral fixed-dose immediate release pharmaceutical compositions comprising the three active pharmaceutical ingredients (APIs) Amlodipine and Atorvastatin or their pharmaceutically acceptable salts or esters, and Candesartan cilexetil with good stability and improved dissolution.

Amlodipine is the international nonproprietary name (INN) of (R*S*)-3-ethyl-5-methyl-2-[(2-aminoethoxy)methyl]-4-(2-chlorophenyl)-6-methyl-1,4-dihydropyridine-3,5-dicarboxylate with the following chemical structure:

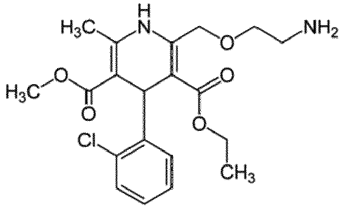

Pharmaceutical compositions of the calcium channel blocker (CCB) Amlodipine are registered and marketed under the trade name NORVASC^{®} for the treatment of, inter alia, hypertension.

Atorvastatin is the INN of (3*R*,5*R*)-7-[2-(4-fluoro-phenyl)-3-phenyl-4-(phenylcarbamoyl)-5-propan-2-ylpyrrol-1-yl]-3,5-dihydroxyheptanoic acid with the following chemical structure:

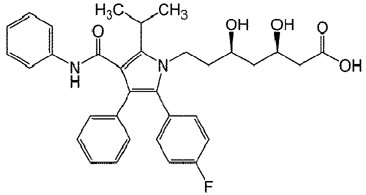

Pharmaceutical compositions of the 3-hydroxy-3-methyl-glutaryl-coenzyme A (HMG-CoA) reductase inhibitor Atorvastatin are registered and marketed under the trade names SORTIS^{®} and LIPITOR^{®} for the treatment of hypercholesterolemia and the prevention of cardiovascular diseases.

Candesartan is the international nonproprietary name (INN) of 2-ethoxy-1-({4-[2-(2*H*-1,2,3,4-tetrazol-5-yl)phenyl]phenyl}methyl)-1*H*-1,3-benzodiazole-7-carboxylic acid which has the following chemical structure:

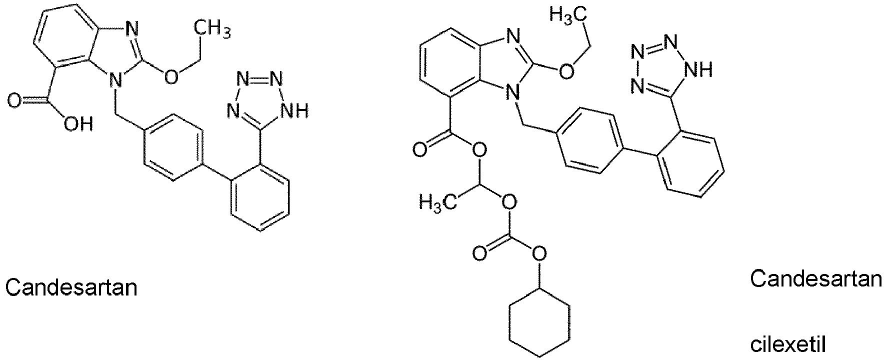

Pharmaceutical compositions of the angiotensin II antagonist Candesartan in the modification of its prodrug Candesartan cilexetil ((±)-1-Hydroxyethyl-2-ethoxy-1-[p-(o-1H-tetrazole-5-ylphenyl)benzyl]-7-benzimidazolecarboxylate)) are registered and marketed under the trade name ATACAND^{®} for the treatment of congestive heart failure, diabetic nephropathies, diabetic retinopathy and hypertension.

As regards fixed-dose pharmaceutical combinations comprising Amlodipine, Atorvastatin and Candesartan cilexetil no such fixed-dose pharmaceutical combination has been registered or marketed yet.

It is desirable to include multiple active ingredients into a single pharmaceutical composition for various reasons, e.g. to provide a single medication to a patient rather than multiple medications. In other words, the inherent advantages of fixed-dose combination therapies result in improved patient compliance because fewer medications have to be consumed by the patient.

The qualitative compositions (excluding coating materials) and strengths of the mono-products NORVASC^{®}, SORTIS^{®} and ATACAND^{®} are displayed in Table 1.

**Table 1**

| **Brand name** | **NORVASC^{®}** | **SORTIS^{®}** | **A TACAND^{®}** |
|---|---|---|---|
| Active pharmaceutical ingredients (APIs) | Amlodipine | Atorvastatin | Candesartan cilexetil |
| Dosage form | Tablet | Film-coated tablet | Tablet |
| Strengths [mg] | 1.25, 2.5, 5, 10 | 10, 20, 40, 80 | 4, 8, 16, 32 |
| Calcium carbonate | - | X | - |
| Microcrystalline cellulose | X | X | - |
| Maize starch | - | - | X |
| Lactose monohydrate | - | X | X |
| Hydroxypropyl cellulose (Hyprolose) | - | X | X |
| Polysorbate 80 | - | X | - |
| Calcium hydrogen phosphate | X | - | - |
| Croscarmellose sodium | - | X | - |
| Carboxymethyl cellulose calcium | - | - | X |
| Sodium starch glycolate | X | - | - |
| Magnesium stearate | X | X | X |

From table 1 it can be concluded that the excipients and the compositions of the mono-products are quite different from each other, as magnesium stearate is the only common excipient. Accordingly, neither the compositions of the marketed mono-compounds can provide a clear guidance how to design, develop and manufacture a uniform and storage stable fixe-dosed oral pharmaceutical composition comprising Amlodipine, Atorvastatin and Candesartan cilexetil which is bioequivalent to NORVASC^{®}, SORTIS^{®} and ATACAND^{®}.

During the development of fixed-dose combinational products comprising two or even more active ingredients, multiple challenges must be overcome. For example, one active ingredient may be incompatible with another API or the stability of one active ingredient may be affected due to its incompatibility with an excipient that is commonly used with another API. In the present case, Amlodipine is incompatible with the excipient lactose, rendering the composition of SORTIS^{®} unsuitable to serve as a basis for a fixed-dose combination of Atorvastatin and Amlodipine.

Amlodipine besilate is highly hygroscopic, sensitive to hydrolysis and to exposure to light. It exhibits physical properties such as low bulk density and high cohesiveness.

Atorvastatin, on the other hand, has been found to be somewhat sensitive to acidic conditions.

Candesartan cilexetil exhibits a low bioavailability and a high tendency to undergo hydrolysis and dealkylation.

Candesartan cilexetil and Amlodipine besilate are susceptible to static electricity, whereas all three APIs exhibit poor flow properties.

All three active substances exhibit quite different solubility profiles.

It has now been found that the fixed-dose pharmaceutical formulations of Amlodipine, Atorvastatin and Candesartan cilexetil according to the present invention satisfy all regulatory requirements as regards stability, processability as well as the dissolution profile of all three active substances compared to their respective brand products.
Figure 1 shows the dissolution of exemplary formulations comprising 5mg Amlodipine besilate, 40mg Atorvastatin calcium and 16mg Candesartan cilexetil prepared according to example 3 after 1- and 3-months storage under accelerated conditions (40°C/75%RH).
Figure 2 shows the dissolution of exemplary formulations comprising 10mg Amlodipine besilate, 40mg Atorvastatin calcium and 32mg Candesartan cilexetil prepared according to example 5 and according to example 6 compared to NORVASC^{®}, SORTIS^{®} and ATACAND^{®}.

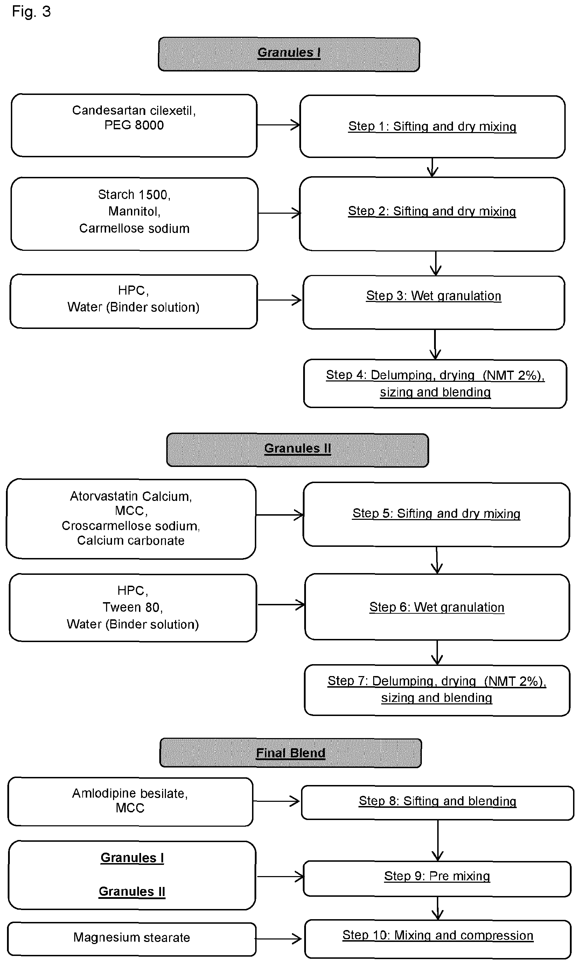

Figure 3 shows a flow chart of the manufacturing process of formulations according to examples 1 to 5.

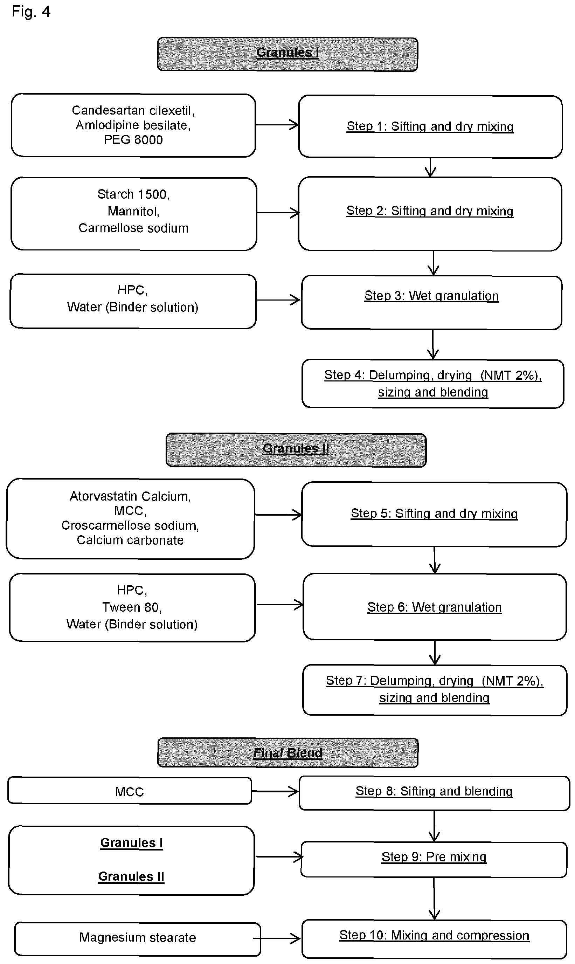

Figure 4 shows a flow chart of the manufacturing process of formulations according to example 6.

The present invention relates to oral fixed-dose immediate release pharmaceutical compositions comprising the active substances Amlodipine, Candesartan cilexetil and Atorvastatin, or their pharmaceutically acceptable salts or esters.

In preferred embodiments of the present invention, the fixed-dose immediate release pharmaceutical compositions are in form of a capsule or a tablet, preferably a monolithic tablet.

In preferred embodiments of the present invention, Amlodipine is utilized in the form of its benzenesulfonate (besilate) salt and Atorvastatin is utilized in the form of its calcium salt.

Amlodipine and its salts such as Amlodipine besilate may be manufactured according to processes known in the art, e.g. as disclosed in patent application EP 0 244 944. Atorvastatin and its salts such as Atorvastatin calcium may be manufactured according to EP 0 247 633 and WO 97/03959. Candesartan cilexetil may be manufactured according to processes known in the art, e.g. as disclosed in patent application EP 0 459 136.

Amlodipine is present in amounts of from 1,25 to 10mg per unit, preferably from 5 to 10mg per unit. Preferably, Amlodipine is present as Amlodipine besilate in amounts from 1.0wt.-% to 5.0wt.-%, based on the total weight of the composition.

Atorvastatin is present in amounts of from 10 to 80mg per unit, preferably from 20 to 40mg per unit. Preferably, Atorvastatin is present in form of its calcium salt in amounts from 5.0wt.-% to 10.0wt.-%, based on the total weight of the composition.

Candesartan cilexetil is present in amounts of from 4 to 32 mg per unit, preferably from 16 to 32mg per unit. Preferably, Candesartan cilexetil is present in amounts of from 2.0wt.-% to 6.0wt.-%, based on the total weight of the composition.

The pharmaceutical compositions of the present invention may comprise excipients such as filler(s), binder(s), lubricant(s), stabilizer(s), solubilizer(s), disintegrant(s), glidant(s) etc.

In the context of the present invention, the terms filler(s), binder(s), lubricant(s), stabilizer(s), solubilizer(s), plasticizer(s), disintegrant(s), glidant(s) etc. shall be understood as including a single compound but also multiple compounds.

Certain pharmaceutical compositions of the present invention may comprise a pharmaceutically acceptable stabilizer for Atorvastatin. Suitable stabilizers for Atorvastatin are alkaline earth metal salts that provide basic conditions such as magnesium carbonate, calcium carbonate, calcium hydroxide, magnesium hydroxide. The ratio of the stabilizer and Atorvastatin is preferably in the range from 5:1 to 1:1 by weight, preferably in the range from 4:1 to 2:1 by weight. The preferred stabilizer for Atorvastatin is calcium carbonate.

Certain pharmaceutical compositions of the present invention may comprise a pharmaceutically acceptable solubilizer for Atorvastatin. Suitable solubilizers for Atorvastatin are ionic or non-ionic surfactants. Examples are sodium lauryl sulfate, poloxamers, lecithins, polyoxyethylenesorbitan fatty acid esters (polysorbates), polyoxyethylated hydrogenated castor oil, polyoxyethylene stearates or mixtures thereof. The ratio of the Atorvastatin solubilizer and Atorvastatin may be in the range from 1:5 to 1:40, preferably from 1:10 to 1:30 by weight, most preferred in the range from 1:15 to 1:25 by weight. The preferred solubilizer is polysorbate 80.

Candesartan cilexetil is somewhat sensitive towards high compression forces. It has been observed that plasticizers such as propylene glycol, polyethylene glycol (PEG), ethanol, glycerin, propylene glycol esters, polyethylene glycol esters and mixtures thereof appear to reduce this sensitivity. Preferred embodiments of the present invention thus comprise plasticizer(s).

As it has been found that high amounts of PEG promote the chemical degradation of Candesartan cilexetil, the amount of PEG should be kept as low as possible. It has been found that PEG in certain ratios of relative to Candesartan cilexetil do provide sufficient physical stabilization but do not cause chemical degradation of Candesartan cilexetil, and furthermore improve the processability of the formulation. As shown below, by applying PEG in amounts of as low as in a ratio of 1:6 to 1:10 (by weight) to Candesartan cilexetil, the formation of degradation products was very moderate even at high compression forces. Accordingly, preferred pharmaceutical formulations according to the present invention comprise PEG in amounts in a ratio of 1:6 to 1:10 (by weight) relative to Candesartan cilexetil.

The pharmaceutical compositions of the present invention may comprise one or more pharmaceutically acceptable filler(s). Pharmaceutically acceptable filler(s) according to the present invention include, but are not limited to cellulose-derived materials like microcrystalline cellulose (mcc), methyl cellulose and ethyl cellulose, starches, sugar alcohols like mannitol, sorbitol or xylitol, acrylate polymers and copolymers; dextrates, dextrin, dextrose, maltodextrin, pectin, gelatin, inorganic diluents like calcium phosphates, calcium silicate, calcium sulfate, kaolin, magnesium oxide and mixtures thereof. Preferred filler(s) are microcrystalline cellulose (mcc), starch, mannitol and mixtures thereof.

Lactose may be used in pharmaceutical compositions when it is physically separated from Amlodipine. Accordingly, one embodiment of the present invention is directed to pharmaceutical compositions comprising lactose which is physically separated from Amlodipine.

The total amount of filler(s) is in the range from 30wt.-% to 90wt.-%, preferably from 40wt.-% to 75wt.-%, based on the total weight of the composition.

The pharmaceutical compositions of the present invention may comprise one or more pharmaceutically acceptable binder(s). Pharmaceutically acceptable binder(s) according to the present invention are hydroxypropyl methylcellulose (HPMC), hydroxypropyl cellulose (HPC), dihydroxypropyl cellulose, hydroxyethyl cellulose, polymethacrylates, sodium alginate, polyvinylpyrrolidone (povidone), pregelatinized starch, vinylpyrrolidone/ vinyl acetate copolymers (copovidone) and mixtures thereof. The total amount of binder(s) is in the range from 0.5wt.-% to 4%wt.-%, preferably in the range of from 1wt.-% to 3%wt.-%, based on the total weight of the composition. Preferred binder is hydroxypropyl cellulose (HPC).

The pharmaceutical compositions of the present invention may comprise one or more pharmaceutically acceptable disintegrant(s). Suitable disintegrant(s) according to the present invention are croscarmellose, carboxymethyl cellulose, crospovidone, sodium starch glycolate, sodium carboxymethyl starch and mixtures thereof. The total amount of disintegrant(s) is in the range from 1wt.-% to 10wt.-%, preferably in the range from 2wt.-% to 8wt.-%, based on the total weight of the composition. Preferred disintegrant(s) are croscarmellose sodium and carboxymethyl cellulose calcium.

The pharmaceutical compositions of the present invention may comprise one or more pharmaceutically acceptable lubricant(s). Suitable lubricants according to the present invention are magnesium stearate, sodium stearyl fumarate, calcium stearate, sodium stearate, stearic acid, hexanedioic acid, hydrogenated vegetable oil, glycerol fumarate and mixtures thereof. The preferred lubricant is magnesium stearate.

The total amount of lubricant(s) is in the range from 0.5wt.-% to 2wt.-%, preferably in the range from 0.7 wt.-% to 1.5 wt.-%, based on the total weight of the composition.

The pharmaceutical compositions according to the present invention may comprise pharmaceutically acceptable glidant(s) such as colloidal silicon dioxide, talcum, magnesium carbonate and mixtures thereof. The amount of glidant(s) is preferably in the range from 0.1wt.-% to 1.5wt.-%, relative to the total weight of the composition.

The invention also relates to processes for the manufacture of storage stable pharmaceutical compositions comprising Amlodipine and Atorvastatin or pharmaceutically acceptable salts or esters thereof and Candesartan cilexetil.

As will be shown in more detail below, one aspect of the present invention relates to pharmaceutical compositions that contain less than 0.5% of impurity D and less than 1.0% of total impurities of Amlodipine after storage for 6 months at 25°C/60%RH.

As will also be shown in more detail below, one aspect of the present invention relates to pharmaceutical compositions that contain less than 0.3% of impurity D, less than 0.3% of impurity H and less than 1.0% of total impurities of Atorvastatin after storage for 6 months at 25°C/60%RH.

As will also be shown in more detail below, one aspect of the present invention relates to pharmaceutical compositions that contain less than 0.5% of each of impurities A, B, E, F and G and less than 1.5% of total impurities of Candesartan cilexetil after storage for at least 6 months at 25°C/60%RH.

As will also be shown in detail below, the oral immediate release fixed-dose pharmaceutical compositions of the present invention show bioequivalence with respect to respect to NORVASC^{®}, SORTIS^{®} and ATACAND^{®}, because they exhibit a significantly improved initial in-vitro dissolution compared to their respective brand products.

The fixed dosed immediate release pharmaceutical compositions according to the present invention may be used in the treatment of hypertension and related diseases.

Further preferred embodiments of the present invention relate to stable oral fixed-dose immediate release pharmaceutical compositions comprising the active substances Amlodipine and Atorvastatin or their respective pharmaceutically acceptable salts or esters, Candesartan cilexetil, one or more pharmaceutically acceptable binder(s), one or more pharmaceutically acceptable filler(s), one or more pharmaceutically acceptable stabilizer(s), one or more pharmaceutically acceptable disintegrant(s), one or more pharmaceutically acceptable lubricant(s) and optionally one or more pharmaceutically acceptable solubilizer(s).

In a further preferred embodiment, the present invention relates to stable oral fixed-dose immediate release pharmaceutical compositions comprising the active substances Amlodipine besilate, Candesartan cilexetil, Atorvastatin calcium, from 30wt.-% to 90wt.-% one or more pharmaceutically acceptable filler(s), from 0.5wt.-% to 4%wt.-% of one or more pharmaceutically acceptable binder(s), one or more pharmaceutically acceptable stabilizer(s), from 3wt.-% to 10wt.-% of one or more pharmaceutically acceptable disintegrant(s), from 0.5wt.-% to 2wt.-% of one or more pharmaceutically acceptable lubricant(s) and optionally one or more pharmaceutically acceptable solubilizer(s), all wt.-% being based on the total weight of the composition.

Further preferred embodiments of the present invention are oral fixed dosed pharmaceutical compositions comprising 5mg/20mg/16mg, 10mg/20mg/16mg, 5mg/40m/16mg, 10mg/40mg/16mg, 5mg/40m/32mg or 10mg/40m/32mg of Amlodipine/Atorvastatin/Candesartan cilexetil for the treatment of hypertension and related diseases.

The pharmaceutical compositions according to the present invention may be manufactured by a process comprising the steps of:
a) preparing a first pre-mix comprising Candesartan cilexetil and a first set of excipients;
b) preparing a second pre-mix comprising Atorvastatin granules and a second set of excipients;
c) combining the pre-mixtures of step a) and step b) with Amlodipine and a third set of excipients; and
d) compressing the mixture of step c) into tablets.

In certain embodiments, Amlodipine is comprised in the first pre-mix instead of being added in step c). Detailed exemplary processes for the manufacture of pharmaceutical compositions according to the present invention are provided in the examples and figures below.

### EXAMPLES

Examples of oral fixed-dose immediate release pharmaceutical compositions according to the present invention are displayed in tables 2a and 2b:

**Table 2a.**

| | **Expl. 1** | | **Expl. 2** | | **Expl. 3** | | **Expl. 4** | |
|---|---|---|---|---|---|---|---|---|
| **Strengths (mg)** | ***5*/*20*/*16*** | | ***10*/*20*/*16*** | | ***5*/*40*/*16*** | | ***10*/*40*/*16*** | |
| | **mg/tab** | **%w/w** | **mg/tab** | **%w/w** | **mg/tab** | **%w/w** | **mg/tab** | **%w/w** |
| **Granules I** | 92.03 | 28.32% | 92.03 | 28.32% | 165.46 | 25.46% | 165.46 | 25.46% |
| **Candesartan cilexetil** | 16.00 | 4.92% | 16.00 | 4.92% | 16.00 | 2.46% | 16.00 | 4.92% |
| **PEG** | 2.60 | 0.80% | 2.60 | 0.80% | 2.60 | 0.40% | 2.60 | 0.40% |
| **Starch (maize)** | 6.84 | 2.10% | 6.84 | 2.10% | 13.68 | 2.10% | 13.68 | 2.10% |
| **Mannitol** | 62.59 | 19.26% | 62.59 | 19.26% | 125.18 | 19.26% | 125.18 | 19.26% |
| **Carboxymethyl cellulose calcium** | 2.75 | 0.85% | 2.75 | 0.85% | 5.50 | 0.85% | 5.50 | 0.85% |
| **HPC** | 1.25 | 0.38% | 1.25 | 0.38% | 2.50 | 0.38% | 2.50 | 0.38% |
| **Granules II** | 125.20 | 38.52% | 125.20 | 38.52% | 250.40 | 38.52% | 250.40 | 38.52% |
| **Atorvastatin calcium** | 21.70 | 6.68% | 21.70 | 6.68% | 43.40 | 6.68% | 43.40 | 6.68% |
| **Microcrystalline Cellulose** | 22.50 | 6.92% | 22.50 | 6.92% | 45.00 | 6.92% | 45.00 | 6.92% |
| **Croscarmellose sodium** | 12.00 | 3.69% | 12.00 | 3.69% | 24.00 | 3.69% | 24.00 | 3.69% |
| **Calcium carbonate** | 65.00 | 20.00% | 65.00 | 20.00% | 130.00 | 20.00% | 130.00 | 20.00% |
| **HPC** | 3.00 | 0.92% | 3.00 | 0.92% | 6.00 | 0.92% | 6.00 | 0.92% |
| **Polysorbate 80** | 1.00 | 0.31% | 1.00 | 0.31% | 2.00 | 0.31% | 2.00 | 0.31% |
| **Extra-Granular** | 107.77 | 33.16% | 107.77 | 33.16% | 234.14 | 36.02% | 234.14 | 36.02% |
| **Amlodipine besilate** | 6.94 | 2.14% | 13.88 | 4.27% | 6.94 | 1.07% | 13.88 | 2.14% |
| **Microcrystalline cellulose** | 97.58 | 30.02% | 90.64 | 27.89% | 220.70 | 33.95% | 213.76.64 | 32.89% |
| **Magnesium stearate** | 3.25 | 1.00% | 3.25 | 1.00% | 6.50 | 1.00% | 6.50 | 1.00% |
| **Total** | **325.00** | **100%** | **325.00** | **100%** | **650.00** | **100.00%** | **650.00** | **100.00%** |

| | | | | | | | | |
|---|---|---|---|---|---|---|---|---|
| * All w/w amounts are wt.-%. based on the weight of the total composition | | | | | | | | |

**Table 2b.**

| | **Expl. 5** | | **Expl. 6** | |
|---|---|---|---|---|
| **Strengths (mg)** | ***10*/*40*/*32*** | | ***10*/*40*/*32*** | |
| | **mg/tab** | **w/w** | **mg/tab** | **w/w** |
| **Granules I** | 184.06 | 28.32% | 197.94 | 30.45% |
| **Candesartan cilexetil** | 32.00 | 4.92% | 32.00 | 4.92% |
| **Amlodipine besilate** | - | - | 13.88 | 2.14% |
| **PEG** | 2.60 | 0.80% | 2.60 | 0.80% |
| **Starch (maize)** | 13.68 | 2.10% | 13.68 | 2.10% |
| **Mannitol** | 125.18 | 19.26% | 125.18 | 19.26% |
| **Carboxymethyl cellulose calcium** | 5.50 | 0.85% | 5.50 | 0.85% |
| **HPC** | 2.50 | 0.38% | 2.50 | 0.38% |
| **Granules II** | 250.40 | 38.52% | 250.40 | 38.53% |
| **Atorvastatin calcium** | 43.40 | 6.68% | 43.40 | 6.68% |
| **Microcrystalline Cellulose** | 45.00 | 6.92% | 45.00 | 6.92% |
| **Croscarmellose sodium** | 24.00 | 3.69% | 24.00 | 3.69% |
| **Calcium carbonate** | 130.00 | 20.00% | 130.00 | 20.00% |
| **HPC** | 6.00 | 0.92% | 6.00 | 0.92% |
| **Polysorbate 80** | 2.00 | 0.31% | 2.00 | 0.31% |
| **Extra-Granular** | 215.54 | 33.16% | 201.66 | 31.02% |
| **Amlodipine besilate** | 13.88 | 2.14% | - | - |
| **Microcrystalline cellulose** | | 30.02% | 195.16 | 30.02% |
| **Magnesium stearate** | 6.50 | 1.00% | 6.50 | 1.00% |
| **Total** | **650.00** | **100%** | **650.00** | **100%** |

The monolithic tablets of examples 1 to 5 may be prepared as follows:
A first premix (Granules I) is prepared by mixing Candesartan cilexetil with PEG, followed by addition of starch, mannitol and carboxymethyl cellulose calcium. The resulting mixture is wet granulated with a HPC binder solution.
A second premix (Granules II) is prepared by mixing Atorvastatin calcium with microcrystalline cellulose, croscarmellose sodium and calcium carbonate and granulating the resulting mixture with a binder solution containing HPC and polysorbate 80. Granules I and II are then mixed with a pre-blend of Amlodipine besilate and MCC and the resulting mixture is blended with magnesium stearate and submitted to tableting.

The preparation of monolithic tablets of example 6 differ in the way that Amlodipine is comprised in the first pre-mix instead of being added in step c).

Exemplary batches of tablets according to example 4 containing 10mg Amlodipine besilate, 40mg Atorvastatin calcium and 16mg Candesartan cilexetil were submitted to storage under accelerated conditions (40°C/75%RH). The respective batches were submitted to dissolution studies under conditions as displayed in table 3.

**Table 3**

| **Dissolution method conditions** | |
|---|---|
| Number of samples (n) | 6 |
| Dissolution volume | 900 mL |
| Paddle speed | 75 rpm¹ |
| Dissolution medium | Phosphate buffer pH 6.5 + 0.35% Tween20 |

| | |
|---|---|
| ¹rpm: rotations per minute | |

The results are displayed in tables 4, 5 and 6.

**Table 4**

| | **Amlodipine (10mg)** | | | | | | | |
|---|---|---|---|---|---|---|---|---|
| **Time Points** | **Initial** | **RSD** | **1M 40/75** | **RSD** | **3M 25/60** | **RSD** | **3M 40/75** | **RSD** |
| **0min** | 0.00 | - | 0.00 | - | 0.00 | - | 0.00 | - |
| **5min** | 85.90 | 4.71% | 85.86 | 3.67% | 77.21 | 5.33% | 86.09 | 2.19% |
| **10min** | 87.26 | 4.15% | 90.74 | 2.19% | 82.14 | 2.90% | 89.71 | 1.89% |
| **15min** | 89.27 | 3.03% | 91.58 | 2.00% | 83.81 | 1.98% | 91.18 | 2.17% |
| **20min** | 90.34 | 3.10% | 91.62 | 1.76% | 85.09 | 1.49% | 90.61 | 2.27% |
| **30min** | 91.22 | 2.76% | 91.99 | 1.62% | 85.42 | 1.23% | 91.35 | 2.33% |
| **45min** | 92.67 | 2.50% | 92.78 | 1.86% | 86.44 | 0.95% | 91.49 | 2.65% |
| **60min** | 93.30 | 3.17% | 96.66 | 1.44% | 95.62 | 0.41% | 95.74 | 1.30% |

**Table 5**

| | **Atorvastatin (40mg)** | | | | | | | |
|---|---|---|---|---|---|---|---|---|
| **Time Points** | **Initial** | **RSD** | **1M 40/75** | **RSD** | **3M 25/60** | **RSD** | **3M 40/75** | **RSD** |
| **0min** | 0.00 | - | 0.00 | - | 0.00 | - | 0.00 | - |
| **5min** | 85.83 | 5.07% | 86.95 | 2.88% | 77.70 | 5.33% | 86.56 | 1.84% |
| **10min** | 87.85 | 4.49% | 92.00 | 1.69% | 83.33 | 2.44% | 90.04 | 1.69% |
| **15min** | 90.37 | 3.80% | 93.30 | 1.51% | 85.23 | 1.65% | 92.20 | 1.92% |
| **20min** | 91.55 | 3.49% | 93.60 | 1.25% | 86.76 | 1.19% | 92.14 | 1.76% |
| **30min** | 92.09 | 3.22% | 94.16 | 1.25% | 87.20 | 1.11% | 92.78 | 2.19% |
| **45min** | 93.42 | 2.85% | 94.97 | 1.63% | 88.50 | 0.88% | 93.08 | 2.29% |
| **60min** | 94.02 | 3.41% | 99.63 | 1.22% | 98.54 | 0.89% | 97.88 | 1.32% |

**Table 6**

| | **Candesartan (16mg)** | | | | | | | |
|---|---|---|---|---|---|---|---|---|
| **Time Points** | **Initial** | **RSD** | **1M 40/75** | **RSD** | **3M 25/60** | **RSD** | **3M 40/75** | **RSD** |
| **0min** | 0.00 | - | 0.00 | - | 0.00 | - | 0.00 | - |
| **5min** | 55.16 | 3.54% | 45.00 | 8.04% | 43.45 | 9.19% | 38.10 | 4.73% |
| **10min** | 65.06 | 3.87% | 63.75 | 4.23% | 59.06 | 5.28% | 50.09 | 2.77% |
| **15min** | 78.99 | 2.60% | 74.05 | 3.22% | 69.63 | 3.74% | 72.00 | 2.25% |
| **20min** | 84.52 | 2.57% | 80.20 | 3.25% | 80.44 | 2.84% | 77.42 | 3.06% |
| **30min** | 88.60 | 2.12% | 86.82 | 3.05% | 83.23 | 2.19% | 82.14 | 2.13% |
| **45min** | 92.51 | 2.13% | 92.89 | 3.77% | 83.77 | 2.38% | 88.70 | 2.00% |
| **60min** | 93.25 | 2.17% | 96.63 | 3.06% | 92.75 | 1.62% | 92.58 | 1.72% |

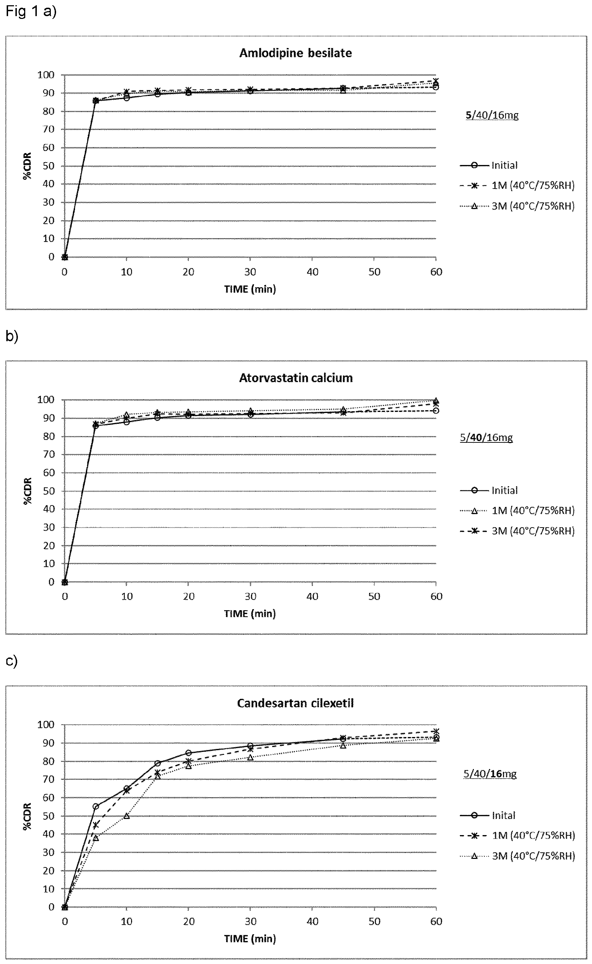

Tables 4, 5 and 6 as well as Fig. 1 a) to c) show that the in-vitro-dissolution profiles for ALL actives do not change significantly after the respective formulations have been submitted to storage under accelerated conditions (40°C/75%RH).

In addition, the assay and related substances of the respective batches were determined. The results are displayed in Tables 7 to 10.

**Table 7**

| Assay | | | | | | |
|---|---|---|---|---|---|---|
| | **Limits** | **Initial** | **1 Month (40°C/75%RH)** | **3 Months (25°C/60%RH)** | **3 Months (40°C/75%RH)** | **6 Months (25°C/60%RH)** |
| **Candesartan** | 95.0 - 105.0 | 102.68 | 100.77 | 100.49 | 100.83 | Ongoing |
| **Amlodipine** | | 98.41 | 96.20 | 97.12 | 96.39 | Ongoing |
| **Atorvastatin** | | 101.92 | 99.33 | 100.44 | 99.88 | Ongoing |

**Table 8**

| Related Substances of Amlodipine (10mg) in % | | | | | | |
|---|---|---|---|---|---|---|
| | Limits | Initial | 1 Month (40°C/75%RH) | 3 Months (25°C/60%RH) | 3 Months (40°C/75%RH) | 6 Months (25°C/60%RH) |
| Impurity D | NMT 0.5 | 0.04 | 0.06 | 0.03 | 0.07 | 0.04 |
| Total impurities | NMT 1.0 | 0.19 | 0.22 | 0.25 | 0.28 | 0.18 |

**Table 9**

| Related Substances of Atorvastatin (40mg) in % | | | | | | |
|---|---|---|---|---|---|---|
| | Limits | Initial | 1 Month (40°C/75%RH) | 3 Months (25°C/60%RH) | 3 Months (40°C/75%RH) | 6 Months (25°C/60%RH) |
| Impurity H | NMT 0.3 | 0.06 | 0.08 | 0.06 | 0.11 | 0.07 |
| Impurity D | NMT 0.3 | 0.05 | 0.06 | 0.04 | 0.06 | 0.08 |
| Total impurities | NMT 1.0 | 0.34 | 0.37 | 0.39 | 0.54 | 0.45 |

**Table 10**

| Related Substances of Candesartan (16mg) in % | | | | | | |
|---|---|---|---|---|---|---|
| | Limits | Initial | 1 Month (40°C/75%RH) | 3 Months (25°C/60%RH) | 3 Months (40°C/75%RH) | 6 Months (25°C/60%RH) |
| Impurity G | NMT 0.5 | ND | 0.01 | ND | 0.01 | ND |
| Impurity A | NMT 0.5 | 0.16 | 0.15 | ND | ND | ND |
| Impurity B | NMT 0.5 | 0.11 | 0.15 | 0.14 | 0.22 | 0.18 |
| Impurity E | NMT 0.5 | 0.05 | 0.06 | 0.05 | 0.07 | 0.06 |
| Impurity F | NMT 0.5 | 0.12 | 0.14 | 0.14 | 0.18 | 0.16 |
| Total impurities | NMT 1.5 | 0.16 | 0.15 | 0.34 | 0.40 | 0.34 |

As can be deducted from tables 7 to 10, all impurity levels remain below their respective limits. The stability experiments indicate that the oral fixed-dose immediate release pharmaceutical compositions are storage stable.

Exemplary batches of tablets according to examples 5 and 6 containing 32mg Candesartan cilexetil were submitted to tableting under different compression forces and then stored for 3 months under accelerated conditions (40°C/75%RH). The results are displayed in table11.

**Table 11**

| Related Substances of Candesartan (32mg) in % | | | | | |
|---|---|---|---|---|---|
| Related substance | Specification | Expl. 5 | | Expl. 6 | |
| | | 85N | 199N | 90N | 210N |
| Impurity G | NMT 0.5 | 0.03 | 0.03 | 0.03 | 0.03 |
| Impurity A | NMT 0.5 | 0.08 | 0.07 | 0.10 | 0.08 |
| Impurity B | NMT 0.5 | 0.33 | 0.40 | 0.35 | 0.42 |
| Impurity E | NMT 0.5 | 0.13 | 0.16 | 0.15 | 0.17 |
| Impurity F | NMT 0.5 | 0.23 | 0.28 | 0.22 | 0.27 |
| Total imp. | NMT 1.5 | 0.56 | 0.84 | 0.57 | 0.86 |

As can be deducted from table 11, the levels of all impurities remain below their respective limits over a wide of range of compression forces, in particular high compression forces of up to more than 200N. Thus, a preferred embodiment of the present invention concerns pharmaceutical compositions of Candesartan cilexetil comprising a plasticizer, characterized in that the composition is storage stable upon applying compression forces from 85N to 210N.

Two test batches of tablets according to Examples 5 and 6, each containing 10mg Amlodipine, 40mg Atorvastatin and 32mg Candesartan were are submitted to in-vitro dissolution studies and compared with their respective reference products (NORVASC^{®}, SORTIS^{®} and ATACAND^{®} under conditions as displayed in table 12.

**Table 12**

| **Dissolution method conditions** | |
|---|---|
| Number of samples (n) | 6 |
| Dissolution volume | 900 mL |
| Paddle speed | 75 rpm¹ |
| Dissolution medium | Phosphate buffer pH 6.5 + 0.70% Tween20 (QC release medium) |

| | |
|---|---|
| ¹rpm: rotations per minute | |

Results are displayed in tables 13 to 15 below.

**Table 13.**

| | Amlodipine (10mg) | | | | | |
|---|---|---|---|---|---|---|
| Time Points | Norvasc | RSD | Expl. 5 | RSD | Expl. 6 | RSD |
| 0min | 0.00 | - | 0.00 | - | 0.00 | - |
| 5min | 64.30 | 4.33% | 86.98 | 2.97% | 92.16 | 1.90% |
| 10min | 80.06 | 2.88% | 92.23 | 1.89% | 94.84 | 1.43% |
| 15min | 88.88 | 2.25% | 93.94 | 1.91% | 95.40 | 1.36% |
| 20min | 91.71 | 1.71% | 94.93 | 2.07% | 95.92 | 1.36% |
| 30min | 96.29 | 1.32% | 96.05 | 2.04% | 96.20 | 1.16% |
| 45min | 97.72 | 1.07% | 97.60 | 1.88% | 96.94 | 1.39% |
| 60min | 98.24 | 1.29% | 99.03 | 1.87% | 97.18 | 1.37% |
| Spin | 99.72 | 1.34% | 100.96 | 1.85% | 98.70 | 1.08% |

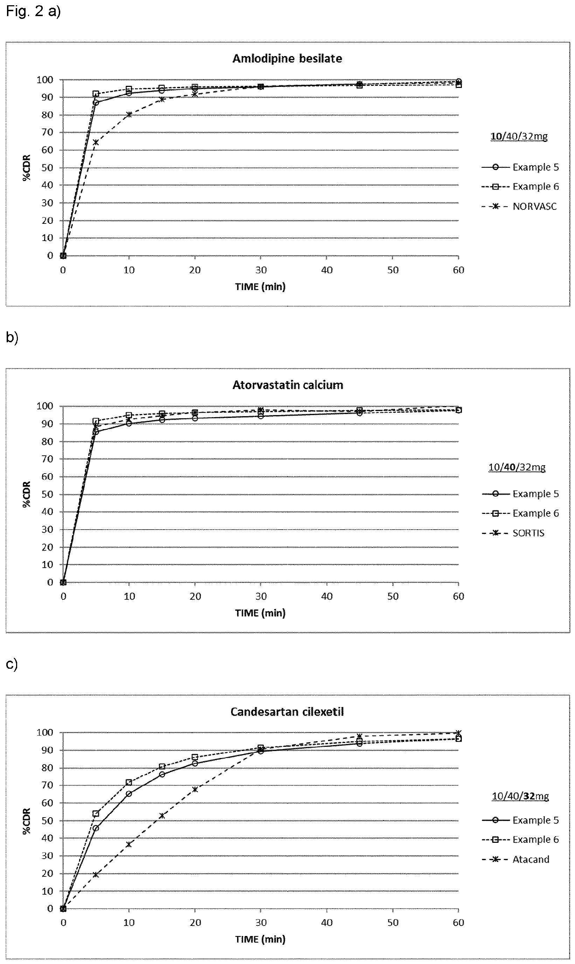

Table 13 and Fig. 2a) indicate that the initial release of Amlodipine is significantly faster compared to the reference product NORVASC^{®}.

**Table 14**

| | Atorvastatin (40 mg) | | | | | |
|---|---|---|---|---|---|---|
| Time Points | SORTIS | RSD | Expl. 5 | RSD | Expl. 6 | RSD |
| 0min | 0.00 | - | 0.00 | - | 0.00 | - |
| 5min | 88.46 | 3.73% | 85.56 | 2.69% | 91.74 | 1.85% |
| 10min | 92.57 | 2.94% | 90.40 | 2.15% | 95.16 | 0.91% |
| 15min | 94.65 | 2.18% | 92.22 | 2.10% | 96.04 | 0.71% |
| 20min | 96.59 | 2.78% | 93.20 | 2.34% | 96.49 | 0.62% |
| 30min | 97.88 | 1.55% | 94.43 | 2.38% | 97.03 | 0.57% |
| 45min | 97.22 | 1.22% | 96.20 | 2.29% | 97.65 | 0.68% |
| 60min | 100.23 | 1.03% | 97.64 | 1.87% | 97.94 | 0.76% |
| Spin | 101.26 | 0.58% | 99.91 | 0.96% | 100.05 | 0.70% |

Table 14 and Fig. 2b indicate that the in-vitro release of Atorvastatin is about equal to the reference product SORTIS^{®}.

**Table 15**

| | Candesartan (32mg) | | | | | |
|---|---|---|---|---|---|---|
| Time Points | ATACAND | RSD | Expl. 5 | RSD | Expl. 6 | RSD |
| 0min | 0.00 | - | 0.00 | - | 0.00 | - |
| 5min | 19.20 | 8.65% | 45.80 | 5.78% | 54.10 | 4.82% |
| 10min | 36.54 | 6.75% | 65.24 | 3.04% | 71.84 | 2.74% |
| 15min | 52.79 | 5.48% | 76.28 | 2.45% | 80.88 | 2.32% |
| 20min | 67.71 | 5.76% | 82.57 | 2.16% | 85.99 | 2.01% |
| 30min | 90.58 | 2.71% | 89.35 | 2.12% | 91.51 | 1.74% |
| 45min | 98.03 | 1.62% | 93.97 | 2.17% | 94.98 | 1.49% |
| 60min | 99.74 | 2.13% | 96.57 | 2.10% | 96.38 | 1.42% |
| Spin | 99.42 | 2.45% | 98.45 | 1.82% | 97.94 | 1.52% |

Table 15 and Fig. 2c indicate that the initial in-vitro-release of Candesartan cilexetil is significantly faster compared to the reference product ATACAND^{®}.

Accordingly, one aspect of the present invention relates to pharmaceutical compositions that exhibit a faster initial in-vitro-release of Candesartan cilexetil compared to ATACAND^{®} and/or a faster initial in-vitro-release of Amlodipine besilate compared to NORVASC^{®}.

Formulations according to example 5 were submitted to an open-label, randomized, single-dose, six-sequences, three-periods crossover bioequivalence study with an at least fourteen-day washout interval between each two consecutive study drug administrations.

The reference products were ATACAND^{®} protect 32 mg tables (32 mg Candesartan cilexetil per tablet, NORVASC^{®} 10 mg tablets (10 mg Amlodipine as Amlodipine besilate per tablet) and SORTIS^{®} 40 mg film tablets (40mg Atorvastatin as Atorvastatin calcium) which were administered concomitantly.

The results of the trial are summarized in Table 16.

**Table 16**

| Pharmacokinetic Parameter | Limits | Amlodipine besilate (10mg) | Atorvastatin calcium (40mg) | Candesartan cilexetil (32mg) |
|---|---|---|---|---|
| Cₘₐₓ | 80.00 - 125.00 | 99.83 | 84.00 | 92.18 |
| AUC_{0→t} | 80.00 - 125.00 | 99.69 | 95.53 | 93.21 |

Table 16 indicates that the ALL actives of the oral fixed-dose combination test preparation containing 10mg Amlodipine besilate, 40mg Atorvastatin calcium and 32mg Candesartan cilexetil are within the general bioequivalence acceptance limits compared to their respective reference products NORVASC^{®}, SORTIS^{®} and ATACAND^{®} and, thus, bioequivalence to the respective reference products can be expected.

Accordingly, one aspect of the present invention relates to oral immediate release fixed-dose pharmaceutical compositions that are bioequivalent with respect to NORVASC^{®}, SORTIS^{®} and ATACAND^{®}.

## Claims

1. A stable oral fixed-dose immediate release pharmaceutical composition comprising the active substances Amlodipine and Atorvastatin or pharmaceutically acceptable salts or esters thereof and Candesartan cilexetil.

2. The stable oral fixed-dose pharmaceutical composition of claim 1, comprising from 5 to 10mg Amlodipine, from 20 to 40mg of Atorvastatin and from 16 to 32mg of Candesartan cilexetil.

3. The stable oral fixed-dose pharmaceutical composition of claim 1 or 2, comprising 5mg/20mg/16mg, 10mg/20mg/16mg, 5mg/40m/16mg, 10mg/40mg/16mg, 5mg/40m/32mg or 10mg/40m/32mg of Amlodipine/Atorvastatin/Candesartan cilexetil for the treatment of hypertension and related diseases.

4. The stable oral fixed-dose pharmaceutical composition of any of the preceding claims, containing less than 0.5% of impurity D and less than 1.0% of total impurities of Amlodipine after storage for 6 months at 25°C/60%RH.

5. The stable oral fixed-dose pharmaceutical composition of any of the preceding claims, containing less than 0.3% of impurity D, less than 0.3% of impurity H and less than 1.0% of total impurities of Atorvastatin after storage for 6 months at 25°C/60%RH.

6. The stable oral fixed-dose pharmaceutical composition of any of the preceding claims, containing less than 0.5% of each of impurities A, B, E, F and G and less than 1.5% of total impurities of Candesartan cilexetil after storage for 6 months at 25°C/60%RH.

7. The stable oral fixed-dose pharmaceutical composition of any of the preceding claims, further comprising a stabilizer for Atorvastatin being selected from the group of magnesium carbonate, calcium carbonate, calcium hydroxide, magnesium hydroxide.

8. The stable oral fixed-dose pharmaceutical composition of claim 7, comprising calcium carbonate in a ratio from 5:1 to 1:1 by weight, compared to the content of Atorvastatin.

9. The stable oral fixed-dose pharmaceutical composition according to any of the preceding claims, further comprising a solubilizer for Atorvastatin being selected from the group of sodium lauryl sulfate, poloxamers, lecithins, polyoxyethylenesorbitan fatty acid esters (polysorbates), polyoxyethylated hydrogenated castor oil, polyoxyethylene stearates.

10. The stable oral fixed-dose pharmaceutical composition of claim 9, comprising polysorbate 80 in a ratio from 1:10 to 1:30 by weight, compared to the content of Atorvastatin.

11. The stable oral fixed-dose pharmaceutical composition of any of the preceding claims, further comprising a plasticizer being selected from the group of propylene glycol, polyethylene glycol (PEG), ethanol, glycerin, propylene glycol esters, polyethylene glycol esters.

12. The stable oral fixed-dose pharmaceutical composition of claim 11, comprising polyethylene glycol (PEG) in a ratio from 1.0:6.0 to 1.0:9.0 by weight, compared to the content of Candesartan cilexetil.

13. The stable oral fixed-dose pharmaceutical composition of any of the preceding claims, further comprising one or more pharmaceutically acceptable filler(s), one or more pharmaceutically acceptable binder(s), one or more pharmaceutically acceptable disintegrant(s) and one or more pharmaceutically acceptable lubricant(s).

14. The stable oral fixed-dose pharmaceutical composition according to claim 13, wherein
a. the amount of filler(s) ranges from 30wt.-% to 90wt.-%.
b. the amount of binder(s) ranges from 0.5 wt.-% to 4 wt.-%.
c. the amount of disintegrant(s) ranges from 1 wt.-% to 10 wt.-%.
d. the amount of lubricant(s) is in the range from 0.5 wt.-% to 2 wt.-%. and
all wt.-%values being relative to the total weight of the composition.

15. A process for the manufacture of a stable oral fixed-dose pharmaceutical composition according to any of the preceding claims, comprising the steps of:
a) preparing a first pre-mix comprising Amlodipine, Candesartan cilexetil and a first set of excipients;
b) preparing a second pre-mix comprising Atorvastatin granules and a second set of excipients;
c) combining the pre- mixtures of step a) and step b) with a third set of excipients;
d) compressing the mixture of step c) into tablets.
